(19) Europäisches Patentamt

European Patent Office

Office européen des brevets

(11) **EP 0 682 002 B1**

(12) **EUROPÄISCHE PATENTSCHRIFT**

(45) Veröffentlichungstag und Bekanntmachung des Hinweises auf die Patenterteilung:
**04.02.1998 Patentblatt 1998/06**

(51) Int. Cl.$^6$: **C07C 29/151**, C07C 31/04

(21) Anmeldenummer: **95104127.6**

(22) Anmeldetag: **21.03.1995**

(54) **Verfahren zur Erzeugung von Methanol**

Method of producing methanol

Procédé pour la préparation de méthanol

(84) Benannte Vertragsstaaten:
**AT BE CH DE DK ES FR GB GR IE IT LI NL PT SE**

(30) Priorität: **10.05.1994 DE 4416425**

(43) Veröffentlichungstag der Anmeldung:
**15.11.1995 Patentblatt 1995/46**

(73) Patentinhaber:
**METALLGESELLSCHAFT AKTIENGESELLSCHAFT**
**60323 Frankfurt am Main (DE)**

(72) Erfinder:
• **Göhna, Hermann**
**D-65812 Bad Soden (DE)**

• **König, Peter, Dr.**
**D-60439 Frankfurt am Main (DE)**

(56) Entgegenhaltungen:
DE-A- 2 705 673          DE-A- 3 518 362
GB-A- 2 203 427

• **DATABASE WPI Week 9323 Derwent Publications Ltd., London, GB; AN 93-186575 & RO-A-102 382 (COMB CHIM VICTORIA)**
• **DATABASE WPI Week 9323 Derwent Publications Ltd., London, GB; AN 93-186574 & RO-A-102 381 (COMB CHIM VICTORIA)**

**Beschreibung**

Die Erfindung betrifft ein Verfahren zur Erzeugung von Methanol aus einem Wasserstoff und Kohlenoxide enthaltenden Synthesegas durch Umsetzung an kupferhaltigen Katalysatoren bei Drücken im Bereich von 20 bis 120 bar und Temperaturen im Bereich von 200 bis 350°C, wobei man das Synthesegas durch einen ersten Synthesereaktor leitet, aus dem ersten Synthesereaktor ein Methanoldampf enthaltendes erstes Produktgemisch abzieht, das erste Produktgemisch kühlt, dabei Methanol kondensiert und von den gasförmigen Bestandteilen des ersten Produktgemisches trennt, wobei man mindestens einen Teil der gasförmigen Bestandteile des ersten Produktgemisches in einen zweiten Synthesereaktor leitet, aus dem man ein Methanoldampf enthaltendes zweites Produktgemisch abzieht, wobei man das zweite Produktgemisch kühlt, dabei Methanol kondensiert und von den gasförmigen Bestandteilen des zweiten Produktgemisches trennt und mindestens einen Teil der gasförmigen Bestandteile des zweiten Produktgemisches in den zweiten Synthesereaktor zurückleitet.

Ein Verfahren dieser Art ist in DE-A-3 518 362 beschrieben. Bei diesem Verfahren benutzt man für den ersten und zweiten Synthesereaktor jeweils einen Schachtreaktor mit einem Katalysatorbett, das durch Wasser indirekt gekühlt wird. Dadurch werden die beiden Reaktoren im wesentlichen isotherm betrieben. Einzelheiten zur Methanolsynthese und zur Erzeugung des Synthesegases sind in Ullmann's Encyclopedia of Industrial Chemistry, 5. Auflage, Band A16, Seiten 467 bis 475 beschrieben, wobei sowohl die Synthese im Röhrenreaktor als auch im adiabatisch betriebenen Schachtreaktor berücksichtigt ist.

Der Erfindung liegt die Aufgabe zugrunde, die Synthese des Methanols aus einem Wasserstoff und Kohlenoxide enthaltenden Synthesegas möglichst kostengünstig durchzuführen und dabei mit einem relativ kleinen Röhrenreaktor auszukommen. Erfindungsgemäß gelingt dies beim eingangs genannten Verfahren dadurch, daß man das Synthesegas mit einer Eintrittstemperatur im Bereich von 220 bis 270°C in den ersten Synthesereaktor leitet, der als Schachtreaktor ausgebildet ist und eine Schüttung eines kupferhaltigen Katalysators enthält, wobei man die Umsetzung im Schachtreaktor adiabatisch und ohne Synthesegas-Kreislauf durchführt, und daß der zweite Synthesereaktor einen in Röhren angeordneten, durch siedendes Wasser indirekt gekühlten kupferhaltigen Katalysator aufweist. Hierbei verzichtet man beim ersten Teil der Synthese, die im Schachtreaktor erfolgt, auf eine Kühlung, die ansonsten bei bekannten Schachtreaktoren entweder durch indirekten Wärmeaustausch mit einem Kühlmedium oder durch Zumischen von kaltem Synthesegas erfolgt. Zweckmäßigerweise sorgt man dafür, daß 10 bis 30 % der dem Schachtreaktor zugeführten Kohlenoxide, d.h. die Summe aus $CO + CO_2$, im Schachtreaktor zu Methanol umgesetzt werden.

Es empfiehlt sich, daß man dem Schachtreaktor ein Synthesegas zuführt, dessen Stöchiometriezahl

$$S = (H_2 - CO_2) : (CO + CO_2)$$

mindestens 1,9 beträgt, welches überwiegend aus Wasserstoff besteht und welches einen CO-Gehalt von 0 bis 20 Vol.-% und einen $CO_2$-Gehalt von 10 bis 25 Vol.-% aufweist, wobei das Volumenverhältnis $CO_2 : CO$ mindestens 1 beträgt. Bei der Methanol-Synthese sind hierbei insbesondere folgende Reaktionen von Bedeutung:

$$CO + 2 H_2 \rightarrow CH_3OH - 90,5 \text{ kJ/mol} \tag{1}$$

$$CO_2 + 3 H_2 \rightarrow CH_3OH + H_2O - 49,4 \text{ kJ/mol} \tag{2}$$

$$CO_2 + H_2 \rightarrow CO + H_2O + 41,1 \text{ kJ/mol} \tag{3}$$

Wenn das Synthesegas nur relativ wenig CO aber relativ viel $CO_2$ enthält, entsteht bei der Synthese im Schachtreaktor, der adiabatisch betrieben wird, zwischen Eintritt und Austritt kein oder nur ein geringer Temperatur-Anstieg. Dadurch ist es möglich, im Schachtreaktor den für die Kupferkatalysatoren günstigen Temperaturbereich von 220 bis etwa 300°C einzuhalten.

Beim erfindungsgemäßen Verfahren wird der größere Teil des Methanols, nämlich üblicherweise 70 bis 90 % der insgesamt im Schachtreaktor und Röhrenreaktor erzeugten Menge, im Röhrenreaktor am indirekt gekühlten Katalysator hergestellt. Dies bedeutet einerseits, daß der aufwendige Röhrenreaktor deutlich kleiner und damit apparativ kostengünstiger hergestellt werden kann, als wenn man die gesamte Methanolmenge nur im Röhrenreaktor erzeugen würde. In bekannter Weise benutzt man im Röhrenreaktor siedendes Wasser als Kühlmittel, wobei Wasserdampf entsteht, der zusätzlich wertvoll ist, weil er unter hohem Druck anfällt. Für das erfindungsgemäße Verfahren ist es nun ein weiterer wichtiger Aspekt, daß die Produktion an Hochdruckdampf im Röhrenreaktor - gegenüber der einstufigen Arbeitsweise ohne vorgeschalteten Schachtreaktor - kaum verringert ist. Der Vorteil der relativ hohen Wasserdampf-Produktion im Röhrenreaktor hängt beim Verfahren der Erfindung damit zusammen, daß im vorgeschalteten Schachtreaktor nur wenig Wärme freigesetzt wird und der weitaus größte Teil des Überschusses an Exothermie erst im Röhrenreaktor wirksam wird.

Um die genannten Vorteile möglichst optimal zu erreichen und die Investitionskosten für eine Produktionsanlage möglichst niedrig zu halten, ist unter anderem darauf zu achten, daß der Schachtreaktor möglichst einfach herzustellen ist. Um an dieser Stelle ohne Kühleinrichtungen auskommen zu können, muß man die bereits erwähnten Kriterien für das Synthesegas einhalten und dabei auch druckabhängige Veränderungen der Reaktionsintensitäten bedenken. Es ist vorteilhaft, dem Schachtreaktor ein Synthesegas mit einem CO-Gehalt von 0 bis 15 Vol.-% zuzuführen, dabei kann das Synthesegas einen relativ hohen $CO_2$-Gehalt von 15 bis 25 Vol.-% aufweisen. Vorteilhafterweise liegt das Volumen-Verhältnis $CO_2$ : CO höher als 2 : 1 und der CO-Gehalt liegt zumeist bei höchstens 10 Vol.-%. Dem Schachtreaktor kann auch ein Synthesegas zugeführt werden, das völlig frei von Kohlenmonoxid ist.

Für die Wahl des Drucks im Schachtreaktor und im Röhrenreaktor bei einer Methanolsynthese-Anlage gibt es zwei generelle Möglichkeiten, nämlich einmal in beiden Reaktoren bei etwa dem gleichen Druck zu arbeiten, so daß nachgeschaltete Anlagenteile gemeinsam verwendet werden können, und die andere Möglichkeit besteht darin, im Schachtreaktor und Röhrenreaktor erheblich unterschiedliche Drücke vorzusehen.

Wenn man den Druck in beiden Reaktoren etwa gleich wählt und einen Druckunterschied von weniger als 5 bar vorsieht, arbeitet man zweckmäßigerweise in beiden Reaktoren im Druckbereich von 60 bis 100 bar und vorzugsweise im Bereich von 70 zu 90 bar. Kann aber der Druckunterschied im Schachtreaktor und Röhrenreaktor größer sein, so empfiehlt es sich, im Schachtreaktor bei einem Druck im Bereich von 20 bis 60 bar und Röhrenreaktor im Druckbereich von 50 zu 100 bar zu arbeiten. Im letztgenannten Fall wird zumeist der Druck im Schachtreaktor um mindestens 7 bar niedriger sein als im Röhrenreaktor.

Für das erfindungsgemäße Verfahren, bei welchem das Synthesegas relativ viel $CO_2$ und relativ wenig CO enthält, empfehlen sich Cu-Zn-Katalysatoren mit einem Atomverhältnis Cu/Zn von 2:1 bis 4:1. Im allgemeinen besteht der Katalysator zu 50 bis 75 Gew.-% aus CuO, zu 15 bis 35 Gew.-% aus ZnO, daneben enthält dieser Katalysatortyp noch 5 bis 20 Gew.-% $Al_2O_3$.

Die vorteilhaften Katalysatoren haben ferner eine spezifische Oberfläche nach BET von mindestens 100 $m^2$/g und dazu eine bestimmte Porenstruktur. Poren mit einem Durchmesser im Bereich von 20 bis 75 Å werden nachfolgend als Mesoporen bezeichnet, Poren mit einem Durchmesser von mehr als 75 Å werden nachstehend als Makroporen bezeichnet (Å = Angström). Im Katalysator liegt der Anteil der Mesoporen zahlenmäßig im Bereich von 20 bis 40 % und der Anteil der Makroporen zahlenmäßig im Bereich von 80 bis 60 %. Der Anteil der Poren mit einem Porendurchmesser von weniger als 20 Å liegt zahlenmäßig bei höchstens 1 %.

Die Herstellung eines Katalysators des bevorzugten Typs geschieht beispielsweise wie folgt:

Eine erste Lösung wird aus 418 g Kupfernitrat, 50 g Zinkoxid, 1,6 l Wasser und 128 g 52,5-%ige $HNO_3$ erzeugt, wobei man diese Lösung mit einem kolloidalen Aluminiummethahydrat-Gel versetzt. Dieses Gel erzeugt man aus einem AlO(OH)-Sol, das bei 50°C unter langsamem Rühren mit 30 g der 52,5-%igen Salpetersäure versetzt wird, um die Aluminiummethahydrat-Teilchen zu peptisieren. Eine zweite Lösung besteht aus 410 g Natriumcarbonat, die in 2 l Wasser gelöst werden. Die beiden Lösungen werden getrennt auf 68°C erwärmt und unter starkem Rühren in der Weise vereinigt, daß der pH-Wert während der Fällung 6,7 beträgt. Der Niederschlag wird unter Rühren bei 68°C eine Stunde lang in der Mutterlauge gealtert. Anschließend wird abfiltriert und mit Wasser natriumfrei gewaschen. Der Filterkuchen wird bei 120°C getrocknet und anschließend 8 Stunden lang bei 280°C kalziniert. Das kalzinierte Produkt wird zerkleinert und nach Zusatz von 2 Gew.-% Graphit zu Tabletten verpreßt. Der so gewonnene Katalysatorvorläufer besteht aus 67,4 Gew.-% CuO, 21,4 Gew.-% ZnO und 11,1 Gew.-% $Al_2O_3$. Das Porenvolumen, gemessen mit der Methode der Quecksilberporosimetrie, beträgt 0,34 ml/g, 42 % der Poren sind Mesoporen und 58 % der Poren sind Makroporen.

Ausgestaltungsmöglichkeiten des Verfahrens werden mit Hilfe der Zeichnung erläutert. Es zeigt:

Fig. 1 eine erste Variante der Verfahrensführung und

Fig. 2 eine zweite Variante der Verfahrensführung.

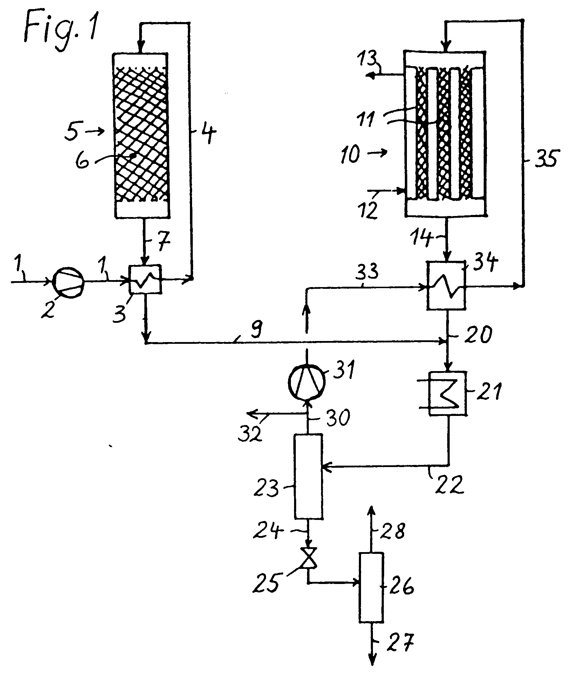

Bei der Verfahrensführung der Fig. 1 wird das durch die Leitung (1) herangeführte, vom Verdichter (2) geförderte Synthesegas im Wärmeaustauscher (3) auf Temperaturen von 220 bis 270°C erhitzt und durch die Leitung (4) dem Schachtreaktor (5) zugeführt. Der Schachtreaktor enthält eine Schüttung (6) eines körnigen Kupferkatalysators. Im Schachtreaktor (5) erfolgt die Umsetzung ohne Kühlung und ohne Kreislaufführung von Synthesegas allein durch einmaliges Hindurchströmen des frischen Synthesegases. Am unteren Ende des Reaktors (5) wird durch die Leitung (7) ein erstes Produktgemisch abgezogen, welches Methanoldampf enthält. Üblicherweise werden im Schachtreaktor 10 bis 30 % der Kohlenoxide (CO + $CO_2$) des Synthesegases der Leitung (4) zu Methanol umgesetzt.

Eine erste Kühlung des Produktgemisches erfolgt im Wärmeaustauscher (3), danach strömt das Produktgemisch durch die Leitung (9), wo es in der Leitung (20) mit dem zweiten Produktgemisch vereinigt wird, das aus dem Röhrenreaktor (10) kommt. Im Kühler (21) werden die beiden Produktgemische so weit gekühlt, daß Methanol kondensiert und das Gemisch strömt dann durch die Leitung (22) zu einem Abscheider (23). Methanolreiches Kondensat zieht man durch die Leitung (24) ab und führt es über ein Entspannungsventil (25) zu einem zweiten Abscheider (26). In der Leitung (27) fällt Rohmethanol an und Restgas verläßt den Abscheider (26) durch die Leitung (28). Das Rohmethanol der Leitung (27) wird einer bekannten, nicht dargestellten destillativen Reinigung zugeführt.

Das am Kopf des Abscheiders (23) abziehende Kreislaufgas wird teilweise durch die Leitung (30) zu einem Verdichter (31) geführt und ein Teil des Gases wird durch die Leitung (32) aus dem Verfahren entfernt, um den Gehalt an Inertgasen zu begrenzen. Das Kreislaufgas, welches die Synthesegas-Komponenten $H_2$, CO und $CO_2$ enthält, wird dann durch die Leitung (33) zunächst zum Wärmeaustauscher (34) geführt und dort auf Temperaturen von etwa 200 bis 250°C erhitzt, bevor es in der Leitung (35) dem Röhrenreaktor (10) aufgegeben wird. In bekannter Weise ist der Röhrenreaktor (10) mit einer großen Anzahl von Röhren (11) ausgestattet, die einen körnigen Kupferkatalysator enthalten. Der Katalysator wird indirekt durch unter hohem Druck siedendes Wasser gekühlt, das man in der Leitung (12) heranführt, der erzeugte Wasserdampf wird in der Leitung (13) abgezogen. Da dieser Wasserdampf etwa unter dem Druck anfällt, der der in den Röhren (11) herrschenden Temperatur als Siedetemperatur entspricht, ist er für die weitere Verwendung von beträchtlichem Wert.

Das im Röhrenreaktor (10) erzeugte Produktgemisch strömt durch die Leitung (14) zum Wärmeaustauscher (34), wo eine erste Kühlung stattfindet und anschließend wird das Produktgemisch mit dem aus der Leitung (9) herangeführten Produktgemisch in der Leitung (20) vereinigt.

Beim Verfahren der Fig. 1 ist der Druck im Schachtreaktor (6) etwa der gleiche wie in den Röhren (11) des Röhrenreaktors (10), wobei man den Druck vorzugsweise um 1 bis 5 bar im Schachtreaktor (6) höher wählt, damit das dort erzeugte Produktgemisch ohne weiteres durch die Leitungen (7) und (9) zur Leitung (20) strömt. In den beiden Reaktoren (6) und (10) herrschen Drücke im Bereich von 20 bis 120 bar und vorzugsweise mindestens 40 bar.

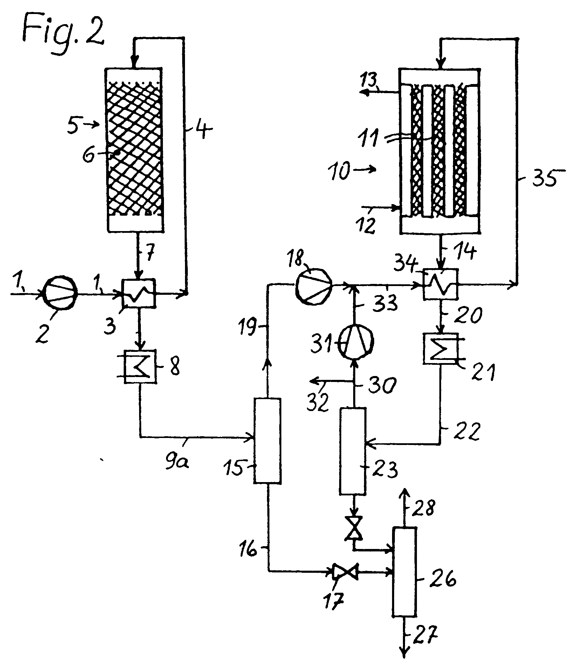

Bei der Verfahrensführung der Fig. 2 arbeitet man im Schachtreaktor (6) und im Röhrenreaktor (10) bei Drücken, die sich erheblich unterscheiden und im Schachtreaktor zumeist um mindestens 7 bar niedriger sind als im Röhrenreaktor. Vorzugsweise liegt der Druck im Schachtreaktor (6) bei 20 bis 60 bar und in den Röhren (11) des Röhrenreaktors (10) bei 50 bis 100 bar. Wegen dieser Druckunterschiede wird das vom Wärmeaustauscher (3) kommende erste Produktgemisch durch einen Kühler (8) geführt, wobei man Methanol kondensiert und das Gemisch gelangt anschließend durch die Leitung (9a) zu einem eigenen Abscheider (15). Methanolreiches Kondensat aus dem Abscheider (15) wird durch die Leitung (16) zunächst zu einem Entspannungsventil (17) geführt und dann in den Abscheider (26) eingeleitet. Restgas, das die Synthesegas-Komponenten $H_2$, CO und $CO_2$ enthält, zieht man mit Hilfe des Verdichters (18) in der Leitung (19) aus dem Abscheider (15) ab und speist es in die Leitung (33) des Kreislaufgases ein, das in der zusammen mit Fig. 1 beschriebenen Weise durch die Leitung (35) in den Röhrenreaktor (10) gelangt. Die weiteren Einzelheiten der Verfahrensführung der Fig. 2 stimmen mit den zusammen mit Fig. 1 erläuterten Einzelheiten überein, wobei Anlagenteile mit übereinstimmenden Bezugsziffern die bereits erläuterte Bedeutung haben.

Beispiel 1

In einer Verfahrensführung gemäß Fig. 1 wird durch die Leitung (1) ein Synthesegas herangeführt, das die in Tabelle I, Spalte A angegebene Zusammensetzung hat:

Tabelle I

|  | A | B |
|---|---|---|
| $CO_2$ | 23,9 Mol-% | 22,1 Mol-% |
| CO | 0,3 Mol-% | 2,2 Mol-% |
| $H_2$ | 73,9 Mol-% | 73,8 Mol-% |
| $CH_4$ | 1,2 Mol-% | 1,2 Mol-% |
| $N_2$ | 0,7 Mol-% | 0,7 Mol-% |

Der im Schachtreaktor (5) und im Röhrenreaktor (10) verwendete Katalysator ist der gleiche, er besteht aus 67,4 Gew.-% CuO, 21,4 Gew.-% ZnO und 11,1 Gew.-% $Al_2O_3$, er wird vor Beginn der Synthese in üblicher Weise reduziert. Der Schachtreaktor enthält 200 kg Katalysator und der Röhrenreaktor enthält 800 kg Katalysator. Im Schachtreaktor arbeitet man bei einem Druck von 80 bar und der Druck in den Röhren des Röhrenreaktors liegt bei 78 bar. Pro Stunde und pro $m^3$ Katalysator führt man dem Schachtreaktor 11000 $Nm^3$ Synthesegas zu.

In verschiedenen Leitungen herrschen folgende, in Tabelle II in der Zeile T1 angegebene Temperaturen:

4

Tabelle II

| Leitung | 4 | 7 | 13 | 35 |
|---|---|---|---|---|
| T1 (°C) | 250 | 286 | 260 | 240 |
| T2 (°C) | 250 | 279 | 260 | 240 |

Das Gas- und Dampfgemisch in der Leitung (7) hat die in der Tabelle III in Spalte A angegebene Zusammensetzung.

Tabelle III

| | A | B | C | D | E |
|---|---|---|---|---|---|
| $CO_2$ (Mol-%) | 18,5 | 14,0 | 11,3 | 19,8 | 9,8 |
| CO (Mol-%) | 3,6 | 3,0 | 2,5 | 5,0 | 2,4 |
| $H_2$ (Mol-%) | 64,5 | 69,1 | 62,0 | 72,9 | 71,1 |
| $CH_4$ (Mol-%) | 1,3 | 8,4 | 9,3 | 1,4 | 10,2 |
| $N_2$ (Mol-%) | 0,8 | 5,1 | 5,6 | 0,8 | 6,3 |
| $CH_3OH$ (Mol-%) | 4,0 | 0,3 | 5,2 | 0,1 | 0,2 |
| $H_2O$ (Mol-%) | 7,3 | 0,1 | 4,1 | - | - |

Dem Röhrenreaktor führt man durch die Leitung (35) ein Gas- und Dampfgemisch in einer Menge von 12000 $Nm^3/m^3/h$ zu, dessen Zusammensetzung in Tabelle III in Spalte B angegeben ist. Das Temperatur-Maximum in den Röhren des Reaktors (10) liegt bei 270°C, bei der Kühlung entsteht Wasserdampf von 48 bar. Das in der Leitung (14) aus dem Röhrenreaktor abgezogene Gas- und Dampfgemisch hat die Zusammensetzung gemäß Spalte C in Tabelle III. In der Leitung (27) fällt ein wasserhaltiges Produktgemisch mit 63,9 Gew.-% Methanol an. Für die Herstellung von 1000 kg dieses Produktgemisches werden durch die Leitung (1) 142 kmol Synthesegas mit der in Tabelle I, Spalte A angegebenen Zusammensetzung gebraucht.

Beispiel 2

In der Verfahrensführung gemäß Fig. 2 wird mit dem gleichen Katalysator und den gleichen Katalysatormengen in den beiden Reaktoren (5) und (10) wie im Beispiel 1 gearbeitet. Die Zusammensetzung des Synthesegases der Leitung (1) ist oben in Tabelle I, Spalte B angegeben, Temperaturen in verschiedenen Leitungen sind obiger Tabelle II der Zeile T2 zu entnehmen. Die dem Schachtreaktor (5) zugeführte Synthesegasmenge beträgt wie im Beispiel 1 11000 $Nm^3/m^3/h$, im Reaktor (5) herrscht ein Druck von 60 bar.

Pro kmol Synthesegas der Leitung (1) zieht man in der Leitung (19) 0,87 kmol Restgas ab, das zusammen mit dem Gas der Leitung (33) auf 100 bar komprimiert in den Röhrenreaktor (10) eintritt. Die Zusammensetzung des Restgases der Leitung (19) zeigt Tabelle III, Spalte D und Spalte E betrifft das Gas der Leitung (35). Das Mengenverhältnis der durch den Verdichter (18) und den Kreislaufverdichter (31) geführten Gasströme beträgt 1:4. Das Produktgemisch der Leitung (27) besteht zu 65,8 Gew.-% aus Methanol, der Rest ist Wasser. Um 1000 kg dieses Produktgemisches zu erzeugen, braucht man 135 kmol Synthesegas der in Tabelle I, Spalte B angegebenen Zusammensetzung.

**Patentansprüche**

1. Verfahren zur Erzeugung von Methanol aus einem Wasserstoff und Kohlenoxide enthaltenden Synthesegas durch Umsetzung an kupferhaltigen Katalysatoren bei Drücken im Bereich von 20 bis 120 bar und Temperaturen im Bereich von 200 bis 350°C, wobei man das Synthesegas durch einen ersten Synthesereaktor (4) leitet, aus dem ersten Synthesereaktor ein Methanoldampf enthaltendes erstes Produktgemisch (7) abzieht, das erste Produktgemisch kühlt, dabei Methanol kondensiert und von den gasförmigen Bestandteilen des ersten Produktgemisches trennt, wobei man mindestens einen Teil der gasförmigen Bestandteile des ersten Produktgemisches in einen zweiten Synthesereaktor (10) leitet, aus dem man ein Methanoldampf enthaltendes zweites Produktgemisch (14) abzieht, wobei man das zweite Produktgemisch kühlt, dabei Methanol kondensiert und von den gasförmigen Bestandteilen des zweiten Produktgemisches trennt und mindestens einen Teil der gasförmigen Bestandteile des zweiten Produktgemisches in den zweiten Synthesereaktor zurückleitet, dadurch gekennzeichnet, daß man das Synthesegas mit einer Eintrittstemperatur im Bereich von 220 bis 270°C in den ersten Synthesereaktor (4) leitet, der als Schachtreaktor ausgebildet ist und eine Schüttung (6) eines kupferhaltigen Katalysators enthält, wobei man die Umsetzung im Schachtreaktor adiabatisch und ohne Synthesegas-Kreislauf durchführt, und daß der zweite Synthesereaktor (10) einen in Röhren (11) angeordneten, durch siedendes Wasser indirekt gekühlten kupferhaltigen Katalysator aufweist.

2. Verfahren nach Anspruch 1, dadurch gekennzeichnet, daß man 10 bis 30 % der Kohlenoxide im Schachtreaktor zu Methanol umsetzt.

3. Verfahren nach Anspruch 1 oder 2, dadurch gekennzeichnet, daß man dem Schachtreaktor ein Synthesegas zuführt, dessen Stöchiometriezahl $S = (H_2 - CO_2) : (CO + CO_2)$ mindestens 1,9 beträgt, welches überwiegend aus Wasserstoff besteht und welches einen CO-Gehalt von 0 bis 20 Vol.-% und einen $CO_2$-Gehalt von 10 bis 25 Vol.-% aufweist, wobei das Volumenverhältnis $CO_2 : CO$ mindestens 1 beträgt.

4. Verfahren nach Anspruch 3, dadurch gekennzeichnet, daß das Synthesegas, das man dem Schachtreaktor zuführt, einen CO-Gehalt von 0 bis 15 Vol.-% aufweist.

5. Verfahren nach Anspruch 3 oder 4, dadurch gekennzeichnet, daß das Synthesegas, das man dem Schachtreaktor zuführt, einen $CO_2$-Gehalt von 15 bis 25 Vol.-% und ein Volumenverhältnis $CO_2 : CO$ von mindestens 2 : 1 aufweist.

6. Verfahren nach einem der Ansprüche 1 bis 5, dadurch gekennzeichnet, daß der Druck im Schachtreaktor und im Röhrenreaktor im Bereich von 60 bis 100 bar liegt, wobei sich die Drücke in den beiden Reaktoren um weniger als 5 bar unterscheiden.

7. Verfahren nach Anspruch 6, dadurch gekennzeichnet, daß der Druck im Schachtreaktor und im Röhrenreaktor im Bereich von 70 bis 90 bar liegt.

8. Verfahren nach einem der Ansprüche 1 bis 5, dadurch gekennzeichnet, daß im Schachtreaktor ein Druck im Bereich von 20 bis 60 bar und im Röhrenreaktor ein Druck im Bereich von 50 bis 100 bar herrscht, wobei der Druck im Schachtreaktor um mindestens 7 bar niedriger ist als im Röhrenreaktor.

9. Verfahren nach Anspruch 1 oder einem der folgenden, dadurch gekennzeichnet, daß man im Schachtreaktor und im Röhrenreaktor einen Katalysator verwendet, der zu 50 bis 75 Gew.-% aus CuO, zu 15 bis 35 Gew.-% aus ZnO und zu 5 bis 20 Gew.-% aus $Al_2O_3$ besteht.

10. Verfahren nach Anspruch 9, dadurch gekennzeichnet, daß der Katalysator ein Cu:Zn-Atomverhältnis von 2:1 bis 4:1 aufweist.

## Claims

1. A method for the production of methanol from a synthesis gas containing hydrogen and carbon oxides by reaction on copper-containing catalysts at pressures in the range of 20 to 120 bar and temperatures in the range of 200 to 350°C, the synthesis gas being passed through a first synthesis reactor (4), a first product mixture (7) containing methanol vapour being withdrawn from the first synthesis reactor, the first product mixture being cooled, in so doing methanol is condensed and separated from the gaseous constituents of the first product mixture, at least part of the gaseous constituents of the first product mixture being passed into a second synthesis reactor (10), from which a second product mixture (14) containing methanol vapour is withdrawn, the second product mixture being cooled, in so doing methanol being condensed and separated from the gaseous constituents of the second product mixture and at least part of the gaseous constituents of the second product mixture being passed back into the second synthesis reactor, characterised in that the synthesis gas is passed at an entry temperature in the range of 220 to 270°C into the first synthesis reactor (4), which is designed as a shaft reactor and contains a bed (6) of a copper-containing catalyst, the reaction in the shaft reactor being performed adiabatically and without circulation of synthesis gas, and that the second synthesis reactor (10) has a copper-containing catalyst indirectly cooled by boiling water and arranged in tubes (11).

2. A method according to Claim 1, characterised in that 10 to 30% of the carbon oxides in the shaft reactor are reacted to form methanol.

3. A method according to Claim 1 or 2, characterised in that a synthesis gas is fed to the shaft reactor which has a stoichiometric number S = ($H_2$ - $CO_2$) : (CO + $CO_2$) of at least 1.9, which consists predominantly of hydrogen and which has a CO content of 0 to 20% by volume and a $CO_2$ content of 10 to 25% by volume, the volume ratio of $CO_2$ : CO being at least 1.

4. A method according to Claim 3, characterised in that the synthesis gas which is supplied to the shaft reactor has a CO content of 0 to 15% by volume.

5. A method according to Claim 3 or 4, characterised in that the synthesis gas which is supplied to the shaft reactor has a $CO_2$ content of 15 to 25% by volume and a volume ratio of $CO_2$ : CO of at least 2 : 1.

6. A method according to one of Claims 1 to 5, characterised in that the pressure in the shaft reactor and in the tubular reactor is in the range of 60 to 100 bar, the pressures in the two rectors differing by less than 5 bar.

7. A method according to Claim 6, characterised in that the pressure in the shaft reactor and in the tubular reactor is in the range of 70 to 90 bar.

8. A method according to one of Claims 1 to 5, characterised in that the pressure in the shaft reactor is in the range of 20 to 60 bar and the pressure in the tubular reactor is in the range of 50 to 100 bar, the pressure in the shaft reactor being at least 7 bar lower than in the tubular reactor.

9. A method according to Claim 1 or one of the following claims, characterised in that a catalyst is used in the shaft reactor and in the tubular reactor which consists of 50 to 75% by weight CuO, 15 to 35% by weight ZnO and 5 to 20% by weight $Al_2O_3$.

10. A method according to Claim 9, characterised in that the catalyst has an atomic ratio of Cu:Zn of 2:1 to 4:1.

**Revendications**

1. Procédé pour la préparation de méthanol à partir d'un gaz de synthèse contenant de l'hydrogène et des oxydes de carbone par mise en réaction sur des catalyseurs cuprifères sous des pressions dans le domaine de 20 à 120 bar et à des températures dans le domaine de 200 à 350°C, dans lequel on guide le gaz de synthèse à travers un premier réacteur de synthèse (4), on extrait du premier réacteur de synthèse un premier mélange de produits (7) contenant de la vapeur de méthanol, on refroidit le premier mélange de produits pour condenser le méthanol et pour le séparer des constituants gazeux du premier mélange de produits, dans lequel on guide au moins une partie des constituants gazeux du premier mélange de produits dans un second réacteur de synthèse (10) à partir duquel on extrait un second mélange de produits (14) contenant de la vapeur de méthanol, dans lequel on refroidit le second mélange de produits pour condenser le méthanol et pour le séparer des constituants gazeux du second mélange de produits, et on recycle au moins une partie des constituants gazeux du second mélange de produits dans le second réacteur de synthèse, caractérisé en ce qu'on guide le gaz de synthèse avec une température d'entrée dans le domaine de 220 à 270°C dans le premier réacteur de synthèse (4) qui est réalisé en forme de cage et qui contient une charge (6) d'un catalyseur cuprifère, dans lequel on effectue la mise en réaction dans le réacteur en forme de cage dans des conditions adiabatiques et sans recyclage du gaz de synthèse, et en ce que le second réacteur de synthèse (10) présente un catalyseur cuprifère disposé dans des tubes (11) et refroidit indirectement via de l'eau en ébullition.

2. Procédé selon la revendication 1, caractérisé en ce qu'on convertit en méthanol 10 à 30 % des oxydes de carbone dans le réacteur en forme de cage.

3. Procédé selon la revendication 1 ou 2, caractérisé en ce qu'on achemine au réacteur en forme de cage un gaz de synthèse dont le nombre stoechiométrique S = ($H_2$ - $CO_2$) : (CO + $CO_2$) s'élève à au moins 1,9, qui est constitué principalement d'hydrogène et qui présente une teneur en CO de 0 à 20% en volume et une teneur en $CO_2$ de 10 à 25% en volume, le rapport volumique $CO_2$ : CO s'élevant à au moins 1.

4. Procédé selon la revendication 3, caractérisé en ce que le gaz de synthèse que l'on achemine au réacteur en forme de cage présente une teneur en CO de 0 à 15% en volume.

5. Procédé selon la revendication 3 ou 4, caractérisé en ce que le gaz de synthèse que l'on achemine au réacteur en forme de cage présente une teneur en $CO_2$ de 15 à 25% en volume et un rapport volumique $CO_2$ : CO d'au moins 2 : 1.

6. Procédé selon l'une quelconque des revendications 1 à 5, caractérisé en ce que la pression régnant dans le réacteur en forme de cage et dans le réacteur tubulaire se situe dans le domaine de 60 à 100 bar, les pressions dans les deux réacteurs différant de moins de 5 bar.

7. Procédé selon la revendication 6, caractérisé en ce que la pression régnant dans le réacteur en forme de cage et dans le réacteur tubulaire se situe dans le domaine de 70 à 90 bar.

8. Procédé selon l'une quelconque des revendications 1 à 5, caractérisé en ce que, dans le réacteur en forme de cage, règne une pression dans le domaine de 20 à 60 bar et, dans le réacteur tubulaire, règne une pression dans le domaine de 50 à 100 bar, la pression régnant dans le réacteur en forme de cage étant d'au moins 7 bar inférieure à celle régnant dans le réacteur tubulaire.

9. Procédé selon la revendication 1 ou selon l'une quelconque des revendications ultérieures, caractérisé en ce qu'on utilise dans le réacteur en forme de cage et dans le réacteur tubulaire, un catalyseur qui est constitué, à concurrence de 50 à 75% en poids, par du CuO, à concurrence de 15 à 35% en poids, par du ZnO et, à concurrence de 5 à 20% en poids, par du $Al_2O_3$.

10. Procédé selon la revendication 9, caractérisé en ce que le catalyseur préSente un rapport atomique Cu : Zn de 2 : 1 à 4 : 1.

EP 0 682 002 B1

## Fig. 1

Fig. 2